# EUROPÄISCHE PATENTSCHRIFT

(11) **EP 0 978 559 B1**
(45) Veröffentlichungstag und Bekanntmachung des Hinweises auf die Patenterteilung: **24.01.2007**
(21) Anmeldenummer: 99114536.8
(22) Anmeldetag: 23.07.1999
(51) Int. Cl.: C12N 5/16, C12N 5/22, A61K 35/12

(54) **Induktion einer Tumorimmunität durch Redirektion von Tumorzellen gegen Antigen-präsentierende Zellen**
Induction of tumour immunity by redirection of tumour cells against antigen presenting cells
Induction d'immunité tumorale par redirection de cellules tumorales contre des cellules présentatrices de l'antigène

(30) Priorität: 06.08.1998 DE 19835633
(43) Veröffentlichungstag der Anmeldung: 09.02.2000
(73) Patentinhaber: GSF-Forschungszentrum für Umwelt und Gesundheit GmbH, 85764 Oberschleissheim (DE)
(72) Erfinder: Mocikat, Ralf, Dr., 82131 Gauting (DE); Lindhofer, Horst, Dr., 82194 Gröbenzell (DE)
(74) Vertreter: Reinhard - Skuhra - Weise & Partner

(56) Entgegenhaltungen:
- WO-A-92/05793
- WO-A-96/30030
- DE-C- 19 634 159
- GOLDSTEIN J. ET AL., : "Cytolytic and cytostatic properties of an anti-human Fc-gamma-RI (CD64) x epidermal growth factor bispecific fusion protein" J.IMMUNOL., Bd. 158, 1997, Seiten 872-879, XP001068992

## Beschreibung

Viele maligne Erkrankungen des Menschen haben trotz der Fortschritte der Chemo- und Radiotherapie der letzten Jahre noch immer eine äußerst ungünstige Prognose. Eine definitive Heilung dieser Erkrankungen ist oft nicht möglich, da nach konventioneller Therapie oft residuelle maligne Zellen im Patienten zurückbleiben, die später zu einem Tumorrezidiv führen. Große Hoffnungen werden dabei auf immuntherapeutische Ansätze gesetzt, mittels derer das Immunsystem des Patienten dazu gebracht wird, den Tumor abzustoßen. Es ist bekannt, daß auf Tumorzellen Tumor-assoziierte Antigene vorkommen und daß das Immunsystem prinzipiell durchaus in der Lage ist, diese zu erkennen und die malignen Zellen anzugreifen. Tumore haben jedoch verschiedene Strategien entwickelt, die es ihnen erlauben, sich der Immunantwort zu entziehen. Dies gelingt ihnen z.B. durch ungenügende Präsentation von Tumor-assoziierten Antigenen und/oder durch unzureichende Aktivierung der in der Regel vorhandenen Tumor-spezifischen T-Zellen. Eine Verstärkung der Antigenpräsentation ist daher ein wünschenswertes Ziel für eine immuntherapeutische Intervention.

In der DE 196 34 159.0 ist ein Verfahren beschrieben, mittels dessen der Immunglobulin-Idiotyp von B-Zell-Lymphomen, der bei dieser Erkrankung ein absolut Tumor-spezifisches Antigen darstellt, gegen professionelle Antigen-präsentierende Zellen (APC) dirigiert wird. Dazu wird durch Fusion der Lymphomzelle mit einem Hybridom, das eine Spezifität gegen APC-Oberflächenmoleküle exprimiert, ein sogenanntes Triom erzeugt. Dieses sezerniert den Tumoridiotyp in Form eines bispezifischen Immunglobulins, welches an die APC bindet. Nach Internalisierung und Prozessierung des Idiotyps werden Idiotyp-abgeleitete Peptide auf der APC dem Immunsystem präsentiert. Dadurch kommt eine effektivere Aktivierung von T-Zellen zustande. Durch die "professionelle" Präsentation des Tumorantigens auf der APC, im Gegensatz zu der unvollständigen Präsentation auf der Tumorzelle, können Tumor-spezifische T-Zellen generiert werden. Wir haben gezeigt, daß für die Induktion eines effektiven Tumorschutzes die Injektion der modifizierten Zellen absolut notwendig ist. Durch Behandlung mit dem gereinigten bispezifischen Immunglobulin konnte keine effiziente Tumorimmunität erzielt werden. Wir führen dies auf eine zusätzliche Immunisierung gegen weitere Tumor-assoziierte Antigene zurück, die nach der Lyse der Triomzellen im behandelten Tier (oder Patienten) zustandekommt. Dieses Verfahren hat allerdings den Nachteil, daß es ausschließlich bei malignen Erkrankungen der B-Zellen anwendbar ist.

Goldstein et al., J. Immunol. 15, 872-9 offenbaren die Herstellung eines bispezifischen Fusionsproteins (Antikörper) gegen EGF-R und FcyRI (CD64). Dieses bispezifische Fusionsprotein wird über einen Expressionsvektor in eine Myelomazellinie eingebracht und exprimiert. Jedoch geht hieraus nicht hervor, daß der Antikörper membranständig ist. Darüber hinaus dient die transfizierte Myelomzellinie lediglich der *in vitro*-Produktion der bispezifischen Fusionsproteine.

DE 196 34 159 betrifft eine Hybridzelle, herstellbar durch Fusion einer aus einem Patienten stammenden malignen B-Zelle und einer Hybridomzelle, welche einen Antikörper produziert, der an ein Oberflächenantigen auf einer Antigen-präsentierenden Zelle bindet. Der Antikörper ist jedoch nicht membranständig sondern wird von der Hybridomzelle sezerniert.

WO 96/30030 beschreibt die Fusion zwischen dendritischen Zellen und Tumorzellen. Eine veränderte menschliche oder tierische Tumorzellen, die einen anti-APC-Antikörper membranständig aufweist, ist hieraus nicht bekannt.

Es ist eine Aufgabe der vorliegenden Erfindung, ein neues Mittel bereitzustellen, um eine Tumorimmunität in einem Patienten zu induzieren, wobei dieses Mittel nicht nur bei B-Zell-Tumoren einsetzbar sein soll.

Diese Aufgabe wird erfindungsgemäß durch eine pharmazeutische Zusammensetzung gelöst, die eine menschliche oder tierische Tumorzelle enthält, die einen membranständigen Antikörper exprimiert, der gegen ein oberflächenantigen auf einer Antigen-exprimierenden Zelle (APC) gerichtet ist, wobei die Tumorzelle kein vom parentalen Tumor hergeleitetes Immunglobulin besitzt, d.h. ursprünglich kein eigenes Immunglobulin in der Tumorzelle vorhanden war. Erst durch die Einführung von Immunglobulin exprimierenden Genen, die für einen Antikörper kodieren, der gegen ein Oberflächenantigen auf einer Antigen-exprimierenden Zelle gerichtet ist, erhalten die Tumorzellen eine spezifische Immunglobulinausstattung.

Mit der erfindungsgemäß verwendeten gentechnologisch abgeänderten Tumorzelle können beliebige Tumorerkrankungen therapiert werden. Beispiele hierfür sind epitheliale Tumoren, beispielsweise Karzinome, mesenchymale Tumoren, bspw. Sarkome sowie hämatopoetische Tumore, bspw. Leukämien und Lymphome. Unter den lymphoproliferativen Erkrankungen können sogar solche der T-Zell-Reihe oder B-Zell-Lymphome, welche kein Immunglobulin produzieren, behandelt werden.

Die erfindungsgemäß verwendete, veränderte Tumorzelle bindet durch den exprimierten anti-APC-Antikörper bevorzugt an Fc-Rezeptoren, Man-nose-5-Rezeptoren oder an MHC-Klasse II-Antigene als Oberflächenantigene.

Als Antigen-präsentierende Zellen dienen insbesondere Monocyten, Makrophagen und dendritische Zellen.

Die erfindungsgemäß bereitgestellte Tumorzelle wird in einer therapeutisch wirksamen Menge zusammen mit verträglichen Träger und/oder Hilfsstoffen, wahlweise nach einer Aufreinigung an den Patienten verabreicht. Das erfindungsgemäß bereitgestellte Arzneimittel, das diese Tumorzelle in einer therapeutisch wirksamen Menge enthält, wird an den am Tumor erkrankten Patienten beispielsweise durch Injektion oder Infusion verabreicht.

In der vorliegenden Erfindung wird gezeigt, daß zur Induktion einer Tumorimmunität ein Immunglobulin-Idiotyp, wie ihn B-Zell-Lymphome besitzen, nicht zwingend erforderlich ist. Das bedeutet, daß das Prinzip einer Redirektion von Tumorantigenen gegen APCs auch auf beliebige andere Tumoren ausdehnbar ist, welche kein Immunglobulin exprimieren. Dies ist ein wichtiger Befund, da die am häufigsten vorkommenden Tumoren des Menschen nicht von der B-Zell-Reihe abgeleitet sind. Es ist ausreichend, wenn die gesamte intakte maligne Zelle in physikalischen Kontakt mit der APC gebracht wird. Wir haben eine Triomvariante generiert, die ihr Tumorimmunglobulin verloren hat, die also nur den Antikörper gegen das APC-Oberflächenmolekül auf ihrer Membran trägt. Diese Variante enthält im Prinzip alle Antigene der parentalen Lymphomzelle (mit Ausnahme des Tumor-abgeleiteten Idiotyps), so daß nach Redirektion der gesamten Zelle an die APC alle Antigene prozessiert und präsentiert werden sollten. Tatsächlich wird nach Immunisierung mit dieser Triomvariante ein letales Inokulum von Wildtyp-Tumorzellen erfolgreich abgestoßen.

Wird dieser Ansatz auf die klinische Situation übertragen, würden maligne Zellen aus einem Patienten, der eine konventionelle Therapie erhält, explantiert, mit einem Antikörper-produzierenden Hybridom fusioniert und in den Patienten zurückgegeben. Alternativ werden die Immunglobulingene, welche für den anti-APC-Antikörper kodieren und welche aus dem Hybridom isoliert wurden, in autologe Tumorzellen transferiert. Die genetisch modifizierten Tumorzellen, die den Antikörper nun auf ihrer Oberfläche exprimieren, werden nach Bestrahlung in den Patienten zurückgegeben. Auf diese Weise wird die gesamte Tumorzelle an die APC herangeführt, was zur Phagozytose und zur Präsentation von Tumorantigenen führen sollte.

Daß die Expression der anti-APC-Spezifität auf der Zelloberfläche für die volle Generierung der Tumorimmunität notwendig ist, konnte in einem weiteren Versuch direkt bewiesen werden, in dem für die Vakzinierung eine Mischung von gereinigtem intakten Triomprotein und einer Triomzell-Variante verwendet wurde, die zwar den Lymphomidiotyp besaß, aber auf Grund des Verlustes der anti-APC-Spezifität nicht mehr an APC's binden konnte. Allein konnten sowohl die Triomzell-Variante als auch das gereinigte lösliche Protein nur einen marginalen Tumorschutz vermitteln. Jedoch zeigte sich, daß durch Mischung der defizienten Zellen mit dem intakten Protein der Antitumor-Effekt von intakten Triomzellen (d.h. 100% Überleben) nicht rekonstituiert werden konnte. Auch dieser Befund unterstreicht die Bedeutung eines direkten physikalischen Kontaktes zwischen Triomzelle und APC.

Nachfolgend wird die Erfindung anhand von Ausführungsbeispielen und einer Abbildung näher beschrieben. Die Erfindung ist jedoch nicht auf diese Ausführungsbeispiele begrenzt, sondern kann im Rahmen der nachfolgenden Ansprüche abgewandelt werden. Weiterhin ist es aufgrund lang andauernder Erfahrungen möglich, die an einem Tiermodell erhaltenen Ergebnisse auch auf den Menschen zu übertragen.

### BEISPIELE:

1. Das aus der BALB/c-Maus stammende B-Zell-Lymphom A20 (ATCC TIB-208) wird mit dem anti-Fc-Rezeptor-Hybridom 2.4G2 (ATCC HB-197) fusioniert. Dazu wird das Hybridom durch Kultur in Gegenwart von 8-Azaguanin HAT-sensitiv gemacht. 5 x 10⁶ Zellen des Fusionspartners werden 30 Minuten in Jodacetamid inkubiert, gewaschen und mit 1,5 x 10⁷ HATsensitiven Zellen gemischt. Die Fusion erfolgt durch zweiminütige Inkubation mit Polyethylenglykol 1500. Die Zellen werden in Mikrotiterplatten ausgebracht und nach zwei bis drei Tagen der Selektion in HAT-Medium zugeführt. Die Hybridzellen werden durch limitierende Verdünnung rekloniert, und eine Variante, die den Lymphomidiotyp verloren hat, wird selektiert. Durch FACS-Analyse wird gezeigt, daß diese Zellen jedoch die 2.4G2-Spezifität noch auf ihrer Oberfläche tragen.
2. BALB/c-Mäusen werden zweimal im Abstand von drei Wochen jeweils 10⁵ Zellen i.p. injiziert, welche die 2.4G2-Spezifität auf der Oberfläche exprimieren. 7 Tage später erfolgt die i.p.-Inokulation mit 10⁵ Wildtyptumor-Zellen (A20). Durch die Präimmunisierung läßt sich eine langdauernde Tumorprotektion erreichen (siehe Abbildung; Gruppe A). In der nicht immunisierten Kontrollgruppe (Gruppe B) führt dieselbe Anzahl von Tumorzellen bei 100% der Tiere zu Tumorwachstum.

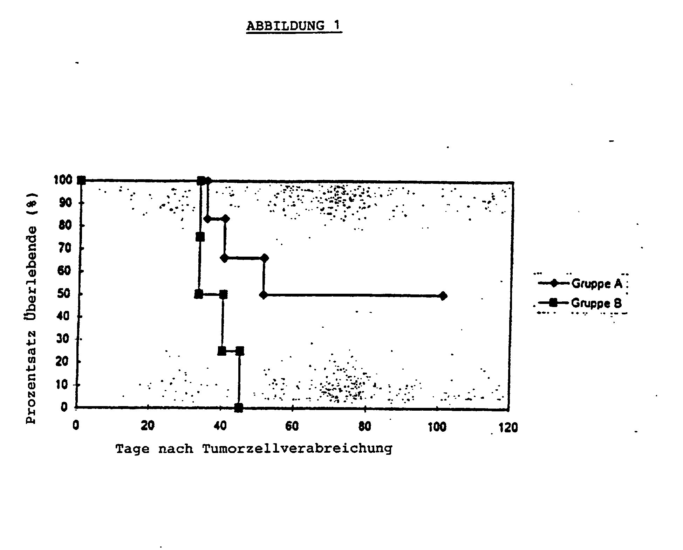

## Patentansprüche

1. Pharmazeutische Zusammensetzung, die eine veränderte menschliche oder tierische Tumorzelle in einer therapeutisch wirksamen Menge zusammen mit einem oder mehreren pharmazeutisch verträglichen Träger- und/oder Hilfsstoffen enthält, wobei die Tumorzelle durch folgendes Verfahren herstellbar ist:
eine aus einem Patienten stammende Tumorzelle wird mit einer Hybridomzelle fusioniert, die einen Antikörper gegen ein Oberflächenantigen auf APCs exprimiert oder die für den anti-APC-Antikörper kodierenden Gene werden derart in die Tumorzelle eingebracht werden, dass diese den anti-APC-Antikörperexprimiert, wobei die Tumorzelle kein vom parentalen Tumor hergeleitetes Immunglobulin exprimiert, und wobei der anti-APC-Antikörper membranständig ist.

2. Pharmazeutische Zusammensetzung nach Anspruch 1,
**dadurch gekennzeichnet,**
**dass** die Tumorzelle eine epitheliale Tumorzelle, eine mesenchymale Tumorzelle oder eine hämatopoetische Tumorzelle ist.

3. Pharmazeutische Zusammensetzung nach Anspruch 2,
**dadurch gekennzeichnet,**
**dass** die hämatopoetische Tumorzelle eine Leukämiezelle oder Lymphomzelle ist, die epitheliale Tumorzelle eine Karzinomzelle ist oder die mesenchymale Tumorzelle eine Sarkomzelle ist

4. Pharmazeutische Zusammensetzung nach einem oder mehreren der vorhergehenden Ansprüche, **dadurch gekennzeichnet,**
**dass** der anti-APC-Antikörper an Fc-Rezeptoren, Mannose-5-Rezeptoren oder an MHC-Klasse II-Antigene als Oberflächenantigene bindet.

5. Verwendung einer pharmazeutischen Zusammensetzung nach einem oder mehreren der Ansprüche 1-4 zur Herstellung eines Arzneimittels zur Tumorbehandlung des Patienten, aus dem die Tumorzelle stammt.

## Claims

1. Pharmaceutical composition containing a modified human or animal tumor cell in a therapeutically effective amount in combination with one or more pharmaceutically acceptable carrier and/or auxiliary substances wherein the tumor cell can be produced by the following method:
- fusing a tumor cell of a patient with a hybridoma cell which does express an antibody against a surface antigen on APCs, or genes encoding said anti-APC antibody are introduced into said tumor cell so that they express said anti-APC antibody wherein said tumor cell does not express an immunoglobulin derived from the parental tumor, and wherein said anti-APC antibody is membrane-associated.

2. Pharmaceutical composition in accordance with claim 1, **characterized in that** said tumor cell is an epithelial tumor cell, a mesenchymal tumor cell or a hematopoietic tumor cell.

3. Pharmaceutical composition in accordance with claim 2, **characterized in that** said hematopoietic tumor cell is a leukemic cell or a lymphoma cell, said epithelial tumor cell is a carcinoma cell or said mesenchymal tumor cell is a sarcoma cell.

4. Pharmaceutical composition in accordance with one or more of the preceding claims, **characterized in that** said anti-APC antibody binds to Fc-receptors, mannose-5 receptors or to MHC class II antigens as surface-associated antigens.

5. Use of a pharmaceutical composition according to one or more of claims 1-4 for the preparation of a medicament for the treatment of tumors of a patient from whom said tumor cell is derived from.

## Revendications

1. Composition pharmaceutique qui contient une cellule tumorale humaine ou animale modifiée en une quantité thérapeutiquement efficace avec un ou plusieurs véhicules et/ou excipients pharmaceutiquement acceptables, la cellule tumorale pouvant être produite par le procédé suivant :
une cellule tumorale provenant d'un patient est fusionnée avec une cellule d'hybridome qui exprime un anticorps dirigé contre un antigène de surface sur des CPA ou les gènes codant pour l'anticorps anti-CPA sont introduits dans la cellule tumorale de telle sorte qu'elle exprime les anticorps anti-CPA, la cellule tumorale n'exprimant pas d'immunoglobuline dérivée de la tumeur parentale, et l'anticorps anti-CPA se trouvant sur la membrane.

2. Composition pharmaceutique selon la revendication 1, **caractérisée en ce que** la cellule tumorale est une cellule tumorale épithéliale, une cellule tumorale mésenchymateuse ou une cellule tumorale hématopoïétique.

3. Composition pharmaceutique selon la revendication 2, **caractérisée en ce que** la cellule tumorale hématopoïétique est une cellule de leucémie ou une cellule de lymphome, la cellule tumorale épithéliale est une cellule de carcinome ou la cellule tumorale mésenchymateuse est une cellule de sarcome.

4. Composition pharmaceutique selon une ou plusieurs des revendications précédentes, **caractérisée en ce que** l'anticorps anti-CPA se lie à des récepteurs Fc, des récepteurs du mannose-5 ou des antigènes de classe II du CMH comme antigènes de surface.

5. Utilisation d'une composition pharmaceutique selon une ou plusieurs des revendications 1 à 4 pour la fabrication d'un médicament pour le traitement d'une tumeur chez le patient dont provient la cellule tumorale.
